(19) Europäisches Patentamt
European Patent Office
Office européen des brevets

(11) EP 4 549 617 A1

(12) EUROPEAN PATENT APPLICATION

(43) Date of publication:
**07.05.2025 Bulletin 2025/19**

(21) Application number: **24207977.0**

(22) Date of filing: **22.10.2024**

(51) International Patent Classification (IPC):
***C25B 1/04*** (2021.01) ***C25B 15/08*** (2006.01)
***C01B 4/00*** (2006.01) ***C07B 59/00*** (2006.01)
***C07C 4/22*** (2006.01) ***C07C 5/05*** (2006.01)
***C07C 7/163*** (2006.01) ***C07C 7/167*** (2006.01)
***C07C 29/17*** (2006.01) ***C07C 41/20*** (2006.01)
***C07C 45/62*** (2006.01) ***C07C 51/36*** (2006.01)
***C07C 211/00*** (2006.01) ***C07D 309/04*** (2006.01)
***C10G 45/00*** (2006.01)

(52) Cooperative Patent Classification (CPC):
(C-Sets available)
**C25B 1/04; C01B 4/00; C07B 59/001; C07C 4/22; C07C 5/05; C07C 7/163; C07C 7/167; C07C 29/17; C07C 29/172; C07C 29/175; C07C 29/177; C07C 41/20; C07C 45/62; C07C 51/36; C07C 211/00;** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.10.2023 EP 23206926**

(71) Applicant: **BASF SE**
**67056 Ludwigshafen am Rhein (DE)**

(72) Inventors:
- **Vossen, Marcus**
  **67117 Limburgerhof (DE)**
- **Stock, Christoph**
  **67056 Ludwigshafen am Rhein (DE)**
- **Ernst, Martin**
  **67056 Ludwigshafen am Rhein (DE)**
- **Harhausen, Sina**
  **67056 Ludwigshafen am Rhein (DE)**
- **Xiong, Jiawen**
  **67056 Ludwigshafen am Rhein (DE)**
- **Hueffer, Stephan**
  **67063 Ludwigshafen (DE)**
- **Krueger, Marco**
  **67056 Ludwigshafen am Rhein (DE)**
- **Weiss, Thomas**
  **67056 Ludwigshafen am Rhein (DE)**

(74) Representative: **BASF IP Association**
**BASF SE**
**GBI - Z078**
**67056 Ludwigshafen (DE)**

(54) **PREPARATION OF SATURATED OR ETHYLENICALLY UNSATURATED (CYCLO)ALIPHATIC COMPOUNDS BY HYDROGENATION OF ETHYLENICALLY OR ACETYLENICALLY UNSATURATED (CYCLO)ALIPHATIC COMPOUNDS USING HYDROGEN WITH LOW DEUTERIUM CONTENT**

(57) A process for the preparation of a saturated or ethylenically unsaturated aliphatic or cycloaliphatic compound comprising the following steps:
(a) providing hydrogen with a molar share of deuterium ≤ 100 ppm, based on the total hydrogen content, by electrolysis of water using electrical power generated at least in part from non-fossil energy,
(b) at least partially hydrogenating an ethylenically unsaturated compound to form the corresponding saturated compound, or at least partially hydrogenating an acetylenically unsaturated compound to form the corresponding saturated or ethylenically unsaturated compound

EP 4 549 617 A1

(52) Cooperative Patent Classification (CPC): (Cont.)
**C07D 309/04; C10G 45/00; C25B 15/081;**
C07B 2200/05; C07C 2601/10; C07C 2601/14;
C07C 2601/20

C-Sets
**C07C 4/22, C07C 13/15;**
**C07C 5/05, C07C 13/12;**
**C07C 7/163, C07C 11/04;**
**C07C 7/163, C07C 11/06;**
**C07C 7/167, C07C 11/04;**
**C07C 7/167, C07C 11/06;**
**C07C 29/17, C07C 33/03;**
**C07C 29/172, C07C 31/20;**
**C07C 29/172, C07C 31/207;**
**C07C 29/172, C07C 35/12;**
**C07C 29/175, C07C 31/125;**
**C07C 29/177, C07C 31/125;**
**C07C 41/20, C07C 43/115;**
**C07C 45/62, C07C 47/02;**
**C07C 45/62, C07C 47/21;**
**C07C 45/62, C07C 49/04;**
**C07C 45/62, C07C 49/213;**
**C07C 45/62, C07C 49/413;**
**C07C 51/36, C07C 53/126;**
**C07C 51/36, C07C 55/10**

## Description

The present invention relates to a hydrogenation process for the preparation of saturated or ethylenically unsaturated aliphatic or cycloaliphatic compounds from the corresponding ethylenically and/or acetylenically unsaturated aliphatic or cycloaliphatic compounds. Ethylenically unsaturated (cyclo)aliphatic compounds have at least one carbon-carbon double bond, while acetylenically unsaturated (cyclo)aliphatic compounds have at least one carbon-carbon triple bond. The obtained saturated or ethylenically unsaturated aliphatic or cycloaliphatic compounds have a lower deuterium content, based on the total hydrogen content, compared to corresponding saturated or ethylenically unsaturated aliphatic or cycloaliphatic compounds generated by reaction with hydrogen that is produced from petrochemical processes using natural gas, condensate or petroleum oil. The invention further relates to the saturated or ethylenically unsaturated aliphatic or cycloaliphatic compounds obtained thereby as well as to their use.

In the preparation of saturated or ethylenically unsaturated aliphatic or cycloaliphatic compounds from the corresponding ethylenically and/or acetylenically unsaturated aliphatic or cycloaliphatic compounds via hydrogenation, hydrogen and in most cases a catalyst is used.

In modern industrial processes, a significant amount of the hydrogen is provided by steam reforming, thus, from natural gas. However, the petrochemical steam reforming process has its negative impacts with regard to its carbon footprint including the consumption of a lot of fossil-based natural resources and energy.

It is therefore an objective of the present invention to provide environmentally friendly saturated or ethylenically unsaturated aliphatic or cycloaliphatic compounds in an environmentally friendly process for making the same, that process using as little fossil-based energy as possible.

The object is achieved by a process for the preparation of a saturated or ethylenically unsaturated aliphatic or cycloaliphatic compound comprising the following steps:

(a) providing hydrogen with a molar share of deuterium $\leq$ 100 ppm, based on the total hydrogen content, by electrolysis of water using electrical power generated at least in part from non-fossil energy,

(b) at least partially hydrogenating an ethylenically unsaturated compound to form the corresponding saturated compound, or at least partially hydrogenating an acetylenically unsaturated compound to form the corresponding saturated or ethylenically unsaturated compound.

The molar share of deuterium in hydrogen and downstream compounds based on hydrogen is given in the present application in ppm, based on the total hydrogen content, which is the mol-ppm content of deuterium, based on the total hydrogen content (in hydrogen or in the compounds discussed, respectively).

The deuterium content of hydrogen and downstream compounds based on hydrogen is given in the present application in atom-ppm based on the total molar hydrogen content (total atoms of protium $^1H$ and deuterium $^2H$). The terms "deuterium content" and "molar share of deuterium" are used synonymously throughout the application.

In physical organic chemistry, a kinetic isotope effect is the change in the reaction rate of a chemical reaction when one of the atoms in the reactants is replaced by one of its isotopes. Formally, it is the ratio of rate constants $k_L / k_H$ for the reactions involving the light ($k_L$) and the heavy ($k_H$) isotopically substituted reactants (isotopologues). This change in reaction rate is a quantum mechanical effect that primarily results from heavier isotopologues having lower vibrational frequencies compared to their lighter counterparts. In most cases, this implies a greater energetic input needed for heavier isotopologues to reach the transition state, and consequently a slower reaction rate.

Isotopic rate changes are most pronounced when the relative mass change is greatest, since the effect is related to vibrational frequencies of the affected bonds. For instance, changing a hydrogen atom (H) to its isotope deuterium (D) represents a 100 % increase in mass, whereas in replacing $^{12}C$ with $^{13}C$, the mass increases by only 8 percent. The rate of a reaction involving a C-H bond is typically 6-10 times faster than the corresponding C-D bond, whereas a $^{12}C$ reaction is only 4 percent faster than the corresponding $^{13}C$ reaction.

A primary kinetic isotope effect may be found when a bond to the isotope atom is being formed or broken. A secondary kinetic isotope effect is observed when no bond to the isotope atom in the reactant is broken or formed. Secondary kinetic isotope effects tend to be much smaller than primary kinetic isotope effects; however, secondary deuterium isotope effects can be as large as 1.4 per deuterium atom.

Step (a)

Step (a) concerns the electrolysis of water using electrical power generated at least in part from non-fossil energy.

Electrolysis of water is an environmentally friendly method for production of hydrogen because it uses renewable $H_2O$ and produces only pure oxygen as by-product. Additionally, water electrolysis utilizes direct current (DC) from sustainable energy resources, for example solar, wind, hydropower and biomass.

It is observed that by electrolysis of water, the deuterium atom content of the hydrogen is lower than in the

hydrogen generated petrochemically, for example as contained in synthesis gas, in general ≤ 100 ppm, preferably in general ≤ 90 ppm, for example from 30 to 75 ppm. The deuterium atom content in electrolytically produced hydrogen may be as low as 10 ppm. The deuterium is mainly present in the form of D-H rather than $D_2$.

One suitable water electrolysis process is alkaline water electrolysis. Hydrogen production by alkaline water electrolysis is a well established technology up to the megawatt range for a commercial level. In alkaline water electrolysis initially at the cathode side two water molecules of alkaline solution (KOH/NaOH) are reduced to one molecule of hydrogen ($H_2$) and two hydroxyl ions (OH-). The produced $H_2$ emanates from the cathode surface in gaseous form and the hydroxyl ions (OH-) migrate under the influence of the electrical field between anode and cathode through the porous diaphragm to the anode, where they are discharged to half a molecule of oxygen ($O_2$) and one molecule of water ($H_2O$). Alkaline electrolysis operates at lower temperatures such as 30-80°C with alkaline aqueous solution (KOH/NaOH) as the electrolyte, the concentration of the electrolyte being about 20% to 30 %. The diaphragm in the middle of the electrolysis cell separates the cathode and anode and also separates the produced gases from their respective electrodes, avoiding the mixing of the produced gases. However, alkaline electrolysis has negative aspects such as limited current densities (below 400 mA/$cm^2$), low operating pressure and low energy efficiency.

In one preferred embodiment of the inventive process, hydrogen is provided by polymer electrolyte membrane water electrolysis. Variants of polymer electrolyte membrane water electrolysis are proton exchange membrane water electrolysis (PEMWE) and anion exchange membrane water electrolysis (AEMWE).

PEM water electrolysis was developed to overcome the drawbacks of alkaline water electrolysis. PEM water electrolysis technology is similar to the PEM fuel cell technology, where solid polysulfonated membranes (Nafion®, fumapem®) are used as an electrolyte (proton conductor). These proton exchange membranes have many advantages such as low gas permeability, high proton conductivity (0.1 ± 0.02 S $cm^{-1}$), low thickness (20-300 μm), and allow high-pressure operation. In terms of sustainability and environmental impact, PEM water electrolysis is one of the most favorable methods for conversion of renewable energy to highly pure hydrogen. PEM water electrolysis has great advantages such as compact design, high current density (above 2 A $cm^{-2}$), high efficiency, fast response, operation at low temperatures (20-80°C) and production of ultrapure hydrogen. The state-of-the-art electrocatalysts for PEM water electrolysis are highly active noble metals such as Pt/Pd for the hydrogen evolution reaction (HER) at the cathode and $IrO_2$/$RuO_2$ for the oxygen evolution reaction (OER) at the anode.

One of the largest advantages of PEM water electrolysis is its ability to operate at high current densities. This can result in reduced operational costs, especially for systems coupled with very dynamic energy sources such as wind and solar power, where sudden spikes in energy output would otherwise result in uncaptured energy. The polymer electrolyte allows the PEM water electrolyzer to operate with a very thin membrane (ca. 100-200 μm) while still allowing high operation pressure, resulting in low ohmic losses, primarily caused by the conduction of protons across the membrane (0.1 S/cm), and a compressed hydrogen output.

The PEM water electrolyzer utilizes a solid polymer electrolyte (SPE) to conduct protons from the anode to the cathode while insulating the electrodes electrically. Under standard conditions the enthalpy required for the formation of water is 285.9 kJ/mol. One portion of the required energy for a sustained electrolysis reaction is supplied by thermal energy and the remainder is supplied through electrical energy.

The half reaction taking place on the anode side of a PEM water electrolyzer is commonly referred to as the Oxygen Evolution Reaction (OER). Here the liquid water reactant is supplied to a catalyst where it is oxidized to oxygen, protons and electrons.

The half reaction taking place on the cathode side of a PEM water electrolyzer is commonly referred to as the Hydrogen Evolution Reaction (HER). Here the protons that have moved through the membrane are reduced to gaseous hydrogen.

PEMs can be made from either pure polymer membranes or from composite membranes, where other materials are embedded in a polymer matrix. One of the most common and commercially available PEM materials is the fluoropolymer PFSA, or Nation®, a DuPont product. While Nation® is an ionomer with a perfluorinated backbone like Teflon, there are many other structural motifs used to make ionomers for proton-exchange membranes. Many use polyaromatic polymers, while others use partially fluorinated polymers.

An overview over hydrogen production by PEM water electrolysis is given in S. Kumar and V. Himabindu, Material Science for Energy Technologies 2 (2019), pp. 4442 - 4454.

An overview over hydrogen production by anion exchange membrane water electrolysis is given in H. A. Miller et al., Sustainable Energy Fuels, 2020, 4, pp. 2114 - 2133.

K. Harada et al., International Journal of Hydrogen Energy 45 (2020), pp. 31389 - 31 395 report a deuterium depletion by a factor from 2 to 3 in polymer electrolyte membrane water electrolysis. The separation factor β

$$\beta = ([H]/[D])_{gas} / ([H]/[D])_{liquid}$$

where "gas" is the evolved gas and "liquid" is water before the electrolysis was found to be between 2 and 3 at current

densities of from 1.0 to 2.0 A $cm^{-2}$, corresponding to a stoichiometric number A of between 4 and 9 at the given water mass flow in the anode. The stoichiometric number A is defined as follows:

$$\lambda = V \times \rho / (J/2F \times 60 \times M_{H2O})$$

where V (mL $min^{-1}$) is the water mass flow in the anode, F is the Faraday constant, J is electrolysis current (A), ρ is the density of water (g mL-1) and $M_{H2O}$ (g $mol^{-1}$) is the molar weight of water. A stoichiometric number A of 10 means that 10 times the amount of fresh water than can be theoretically consumed by electrolysis at the given electrolysis current is supplied to the anode.

H. Sato et al., International Journal of Hydrogen Energy 46 (2021), pp. 33 689 - 33 695, report for anion exchange membrane water electrolysis that deuterium concentration in the evolving hydrogen gas is diluted by approximately 1/5 against the feed water, at A = 4.

Hence, deuterium in the evolving hydrogen gas can easily be depleted by a factor of from 2 to 5 with regard to feed water in polymer electrolyte membrane water electrolysis. Depending on the electrolysis conditions (water flow, current density), even higher depletion factors are possible. Since the average deuterium content of water is about 150 ppm, based on the total hydrogen content, hydrogen provided in step (a) of the inventive process may have a deuterium content of from 30 to 75 ppm, based on the total hydrogen content, or even lower.

The electrical power is generated at least in part from non-fossil, renewable resources. In other words, part of the electrical power can still be produced from fossil fuels, preferably from natural gas, since combustion of natural gas causes much lower carbon dioxide emission per Megajoule of electrical energy produced than combustion of coal. However, the portion of electrical energy produced from fossil fuels should be as low as possible, preferably ≤ 50%, preferably ≤ 30%, most preferably ≤ 20%.

The electrical power from non-fossil resources used in water electrolysis according to the invention can be generated by nuclear energy. Nuclear energy is considered renewable by the European Commission, as long as certain preconditions (i. a. safe long-term storage of nuclear waste) are fulfilled.

The electrical power from non-fossil resources used in water electrolysis according to the invention is preferably generated from wind power, solar energy, biomass, hydropower and geothermal energy.

In one preferred embodiment of the inventive process, the electrical power used in water electrolysis is generated from hydropower. There are many forms of hydropower. Traditionally, hydroelectric power comes from constructing large hydroelectric dams and reservoirs. Small hydro systems are hydroelectric power installations that typically produce up to 50 MW of power. They are often used on small rivers or as a low-impact development on larger rivers. Run-of-the-river hydroelectricity plants derive energy from rivers without the creation of a large reservoir. The water is typically conveyed along the side of the river valley (using channels, pipes and/or tunnels) until it is high above the valley floor, whereupon it can be allowed to fall through a penstock to drive a turbine.

Wave power, which captures the energy of ocean surface waves, and tidal power, converting the energy of tides, are two forms of hydropower with future potential.

In one further preferred embodiment of the inventive process, the electrical power used in electrolysis is generated at least in part from geothermal energy. Geothermal energy is the heat that comes from the sub-surface of the earth. It is contained in the rocks and fluids beneath the earth's crust and can be found as far down to the earth's hot molten rock, magma.

To produce power from geothermal energy, wells are dug a mile deep into underground reservoirs to access the steam and hot water there, which can then be used to drive turbines connected to electricity generators. There are three types of geothermal power plants; dry steam, flash and binary. Dry steam is the oldest form of geothermal technology and takes steam out of the ground and uses it to directly drive a turbine. Flash plants use high-pressure hot water into cool, low-pressure water whilst binary plants pass hot water through a secondary liquid with a lower boiling point, which turns to vapor to drive the turbine.

In one further preferred embodiment of the inventive process, the electrical power used in water electrolysis is generated from wind power. Wind power can be used to run wind turbines. Modern utility-scale wind turbines range from around 600 kW to 9 MW of rated power. The power available from the wind is a function of the cube of the wind speed, so as wind speed increases, power output increases up to the maximum output for the particular turbine. Areas where winds are stronger and more constant, such as offshore and high-altitude sites, are preferred locations for wind farms.

In one further preferred embodiment of the inventive process, the electrical power used in water electrolysis is generated from solar power, particularly preferred from photovoltaic systems. A photovoltaic system converts light into electrical direct current (DC) by taking advantage of the photoelectric effect. Concentrated solar power (CSP) systems use lenses or mirrors and tracking systems to focus a large area of sunlight into a small beam. CSP-Stirling has by far the highest efficiency among all solar energy technologies.

In one further preferred embodiment of the inventive process, the electrical power used in water electrolysis is generated from biomass. Biomass is biological material derived from living, or recently living organisms. It most often

refers to plants or plant-derived materials which are specifically called lignocellulosic biomass. As an energy source, biomass can either be used directly via combustion to produce heat or electricity, or indirectly after converting it to various forms of biofuel. Conversion of biomass to biofuel can be achieved by different methods which are broadly classified into: thermal, chemical, and biochemical methods. Wood was the largest biomass energy source as of 2012; examples include forest residues - such as dead trees, branches and tree stumps -, yard clippings, wood chips and even municipal solid waste. Industrial biomass can be grown from numerous types of plants, including miscanthus, switchgrass, hemp, corn, poplar, willow, sorghum, sugarcane, bamboo, and a variety of tree species, ranging from eucalyptus to oil palm (palm oil).

Plant energy is produced by crops specifically grown for use as fuel that offer high biomass output per hectare with low input energy. The grain can be used for liquid transportation fuels while the straw can be burned to produce heat or electricity. Biomass can be converted to other usable forms of energy such as methane gas or transportation fuels such as ethanol and biodiesel. Rotting garbage, and agricultural and human waste, all release methane gas - also called landfill gas or biogas. Crops, such as corn and sugarcane, can be fermented to produce the transportation fuel, ethanol. Biodiesel, another transportation fuel, can be produced from left-over food products such as vegetable oils and animal fats.

Step (b)

In step (b), an ethylenically and/or acetylenically unsaturated aliphatic or cycloaliphatic compound is hydrogenated using the hydrogen provided in step (a) to form the corresponding saturated or ethylenically unsaturated aliphatic or cycloaliphatic compound.

Preferred ethylenically and/or acetylenically unsaturated aliphatic or cycloaliphatic compounds to be hydrogenated in step (b) contain a total of from 2 to 20 carbon atoms and from 1 to 5, preferably from 1 to 3 carbon-carbon double and/or carbon-carbon triple bonds. The compounds can also contain aromatic groups, in particular one (optionally substituted) phenyl group. Aromatic groups will not be hydrogenated in the inventive process.

The ethylenically and/or acetylenically unsaturated aliphatic or cycloaliphatic compounds to be hydrogenated in step (b) can contain from 1 to 5, preferably from 1 to 3 functional groups selected from hydroxyl groups, carbonyl groups, nitrile groups, ether groups and amino groups. Carbonyl groups can be keto groups or can be part of aldehyde groups, carboxylic acid groups, carboxylic acid ester groups and dicarboxylic acid anhydride groups. Carbonyl groups, in particular aldehyde groups, may also be hydrogenated in the process of the invention.

Cycloaliphatic compounds can contain ring heteroatoms, preferably selected from nitrogen, oxygen and sulfur atoms, preferably nitrogen and oxygen atoms, most preferably oxygen atoms. Preferred cycloaliphatic compounds contain 5, 6, 7, 8 or 12 ring carbon atoms, which can be replaced by 1 to 3, in particular 1 or 2 ring heteroatoms, preferably nitrogen or oxygen atoms.

In certain embodiments of the inventive process, an ethylenically unsaturated aliphatic or cycloaliphatic compound containing two carbon-carbon double bonds is partially hydrogenated to an ethylenically unsaturated aliphatic or cycloaliphatic compound containing only one carbon-carbon double bond.

In other embodiments of the inventive process, an acetylenically unsaturated aliphatic or cycloaliphatic compound is partially hydrogenated to the corresponding ethylenically unsaturated aliphatic or cycloaliphatic compound.

The process of hydrogenating ethylenically and/or acetylenically unsaturated aliphatic or cycloaliphatic compounds into saturated or ethylenically unsaturated aliphatic or cycloaliphatic compound can be carried out in various ways, which can be differentiated according to the type of catalyst, the feedstock and the physical state of the reactants.

Hydrogenation of Carbon-carbon double and triple bonds is widely discussed in the literature - see March's Advanced Organic Chemistry: Reactions, Mechanisms and Structure, 8th edition Chapter "Addition to Carbon-carbon Multiple bonds", Wiley, February 2020 for reference and C.Stock (2023) Hydrogenation and Dehydrogenation; in Ullmann's Encyclopedia of Industrial Chemistry (https://doi.org/10.1002/14356007.a13_487.pub3).

Most carbon-carbon double bonds, whether substituted by electron-donating or electron-withdrawing substituents, can be catalytically hydrogenated, usually in quantitative or near quantitative yields as described for example in Rylander "Catalytic Hydrogenation in Organic Synthesis" Academic Press, New York 1979. Almost all known alkenes add hydrogen at temperatures between 0 and 275 degree Celsius. Many functional groups may be present in the molecule, e.g. OH, COOH, NH2, CHO, COR, COOR and CN. Some of these groups are also susceptible to catalytic reduction, but it is usually possible to find conditions under which double bonds can be reduced selectively (see Rylander, "Catalytic Hydrogenation over Platinum Metals", Academic Press 1967, pp. 59-120).

Catalysts are usually classified into two broad classes: homogeneous and heterogeneous catalysts. Homogeneous catalysts dissolve in the solvent that contains the substrate. Heterogeneous catalysts are solids that are suspended in the same solvent with the substrate or are treated with gaseous substrate. A review of catalysts used for hydrogenation is given in C.Stock (2023) Hydrogenation and Dehydrogenation; Chapter 1.3: "Catalysts" in Ullmann's Encyclopedia of Industrial Chemistry (https://doi.org/10.1002/14356007.a13_487.pub3).

Hydrogenations in many cases are carried out at room temperature and just above atmospheric pressure, but some double bonds are more resistant and require considerably higher temperatures and pressures. The resistance is

usually a function of increasing substitution and is presumably caused by steric effects. Trisubstituted double bonds may require 25°C and 100 atm, while tetrasubstituted double bonds require 275°C and 100 atm.

Most preferred are the following hydrogenations (1) to (26).

(1) Hydrogenation of Butynediol to butanediol

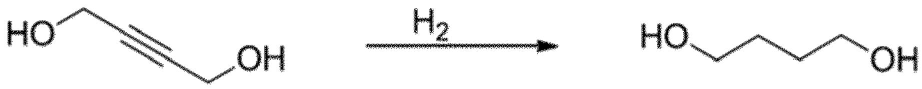


The hydrogenation can be carried out as continuous fixed bed process on a nickel catalyst at up to 180°C und 280 bar. In the hydrogenation, tetrahydrofuran can be formed in part. Hydrogenation in the gas phase is also possible. Suitable hydrogenation processes are for instance described in US 5068468, EP 0177912 and WO 2012/095777.

(2) Hydrogenation of dimethylhexynediol to give dimethylhexandiol:

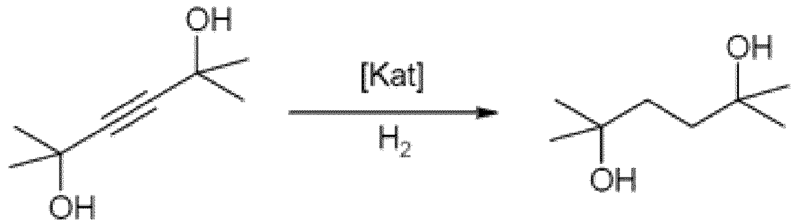


The hydrogenation can be carried out at 50 to 100°C and 20 to 50 bar on a (preferably on $Al_2O_3$) supported palladium catalyst. Suitable hydrogenation processes are for example described in DE 10351396, US 3371121, EP 0333296 and DE 19500479.

(3) Hydrogenation of maleic acid derivatives to give succinic acid derivatives:

The hydrogenation of maleic acid esters or maleic acid anhydrides can be carried out on supported palladium or platinum catalysts. Hydrogenation is also possible in the gas phase (Kvaerner Process). Suitable hydrogenation processes are for example described in WO 2021/115813, WO 97/43242 and WO 99/25678.

(4) Hydrogenation of cyclododecadienone to give cyclododecanone:

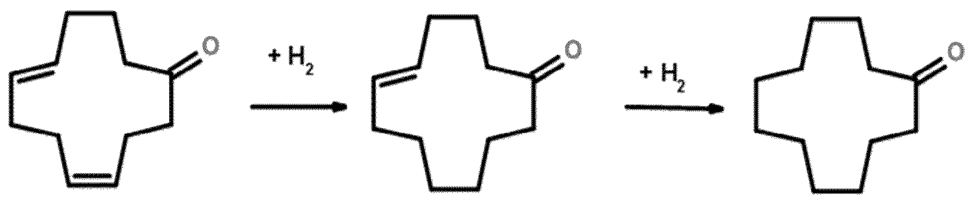


The hydrogenation can be carried out as fixed bed process on palladium on aluminum oxide at 100 to 200°C and 30 bar. Suitable hydrogenation processes are for example described in WO 2005/030690, WO 2008/000757 and WO 2007/006789.

(5) Hydrogenation of 2-ethylhexenal to give 2-ethylhexanal:

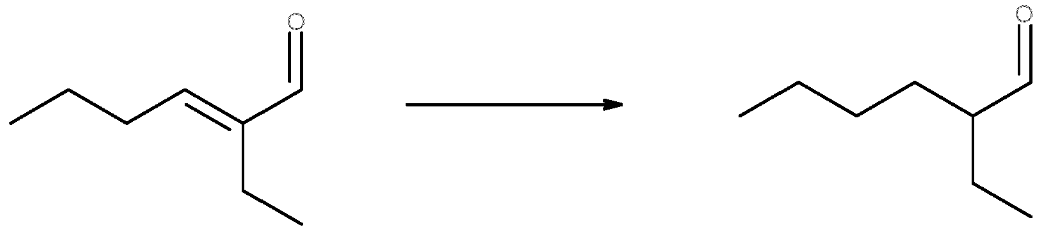

The selective partial hydrogenation can be carried out as continuous fixed bed process on supported palladium catalysts at 70 to 150°C and 10 to 50 bar. A suitable hydrogenation process is described in Favre-Réguillion, Organic Process Research and Development, 2016, 20, 90-94.

(6) Hydrogenation of 2-ethylhexenal to give 2-ethylhexanol:

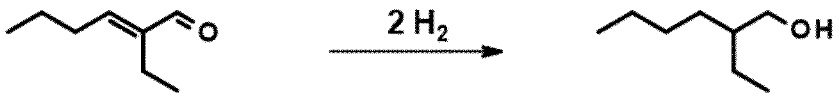


Both the double bond and the aldehyde group are hydrogenated. The hydrogenation can be carried out as continuous fixed bed hydrogenation on copper or nickel catalysts. The hydrogenation can be carried out in the gas phase or liquid phase. High pressure and low pressure variants exist. Typical conditions are 90 to 150°C and 20 to 40 bar in the liquid phase. Suitable hydrogenation processes are for example described in DE 3530839 and US 2013/0237726.

(7) Hydrogenation of 2-propylheptenal and 4-Methyl-2-propylhexenal to give 2-propylheptanol and 4-Methyl-2-propylhexanol:

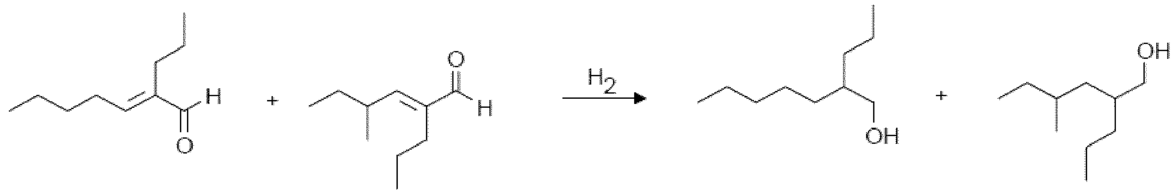


The hydrogenation can be carried out as gas phase or liquid phase process on copper- or nickel-based catalysts. Suitable hydrogenation processes are described in CN 116041142, Tang et al., Applied Catalysis A, General 273, 2004, 171-176, DE 3530839 and US 2013/0237726.

(8) Partial and selective hydrogenations of cracker fractions:

Removal from alkynes and dienes from cracker fractions can be carried out on supported palladium catalysts. Low boiling fractions can be hydrogenated in the gas phase, higher boiling fractions are hydrogenated in the liquid phase on a fixed bed catalyst.

Removal of propyne and propadiene from a $C_3$ feed stream by hydrogenation and removal of acetylene from ethylene feed streams by hydrogenation are for example described in EP 0011906, EP 0885273, A. Borodzinski, G.C., Bond Catalysis Reviews Volume 48, 2006, 91-144.

Removal of dienes from cracker streams are for example described in WO 2019/081628 and DE 10259858.

Hydrogenation of $C_5^+$-fractions is for example described in US 3459657, DE 3709328 and DE 102007037785.

(9) Hydrogenation of citral to give citronellal:

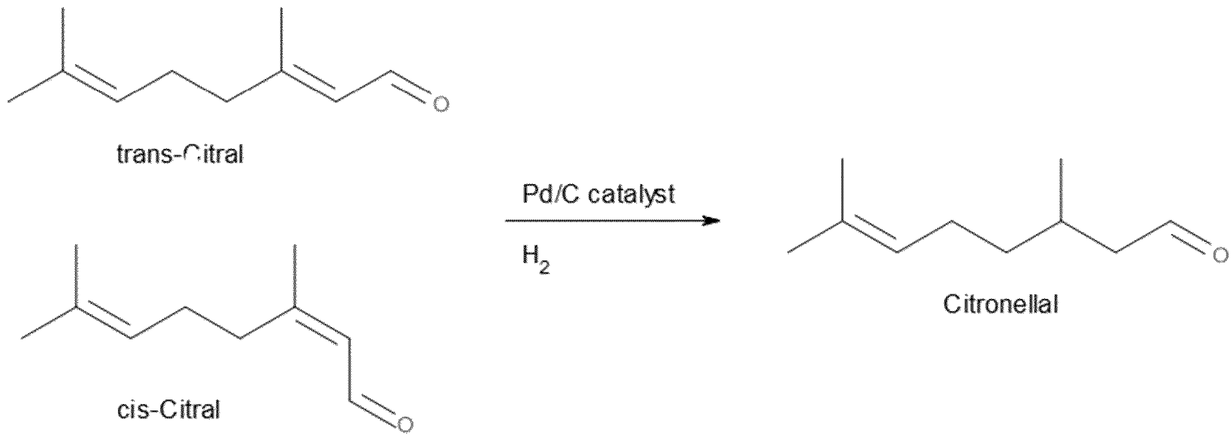


trans-Citral

cis-Citral

Citronellal

The hydrogenation can be carried out on a Pd/C catalyst in suspension. Typical conditions are a maximum pressure of 10 bar and 70 to 90 °C. Methanol and additives can be added. Suitable hydrogenation processes are described in DE 2839474 and DE 19814879. Of particular importance is the homogeneous enantio-selective hydrogenation of cis-citral (Menthol process), as described in WO 2014/167014 and US 2010/0249467.

(10) Hydrogenation of isopulegol to give menthol:

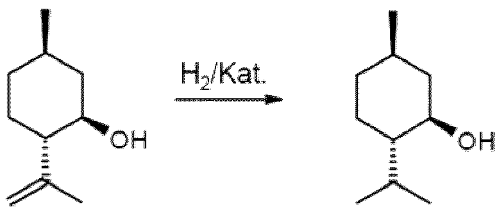


L-Isopulegol L-Menthol

The hydrogenation (to yield the racemate or the pure enantiomer) can be carried out on nickel-based catalysts, as described for example in WO 2009/013192.

(11) Preparation of 3,7-Dimethyloctan-1-ol by hydrogenation of (E)-3,7-dimethylocta-3,6-dien-1-ol (isonerol 1), (Z)-3,7-dimethylocta-3,6-dien-1-ol (isonerol 2) and 3,7-dimethyloct-6-en-1-ol (citronellol):

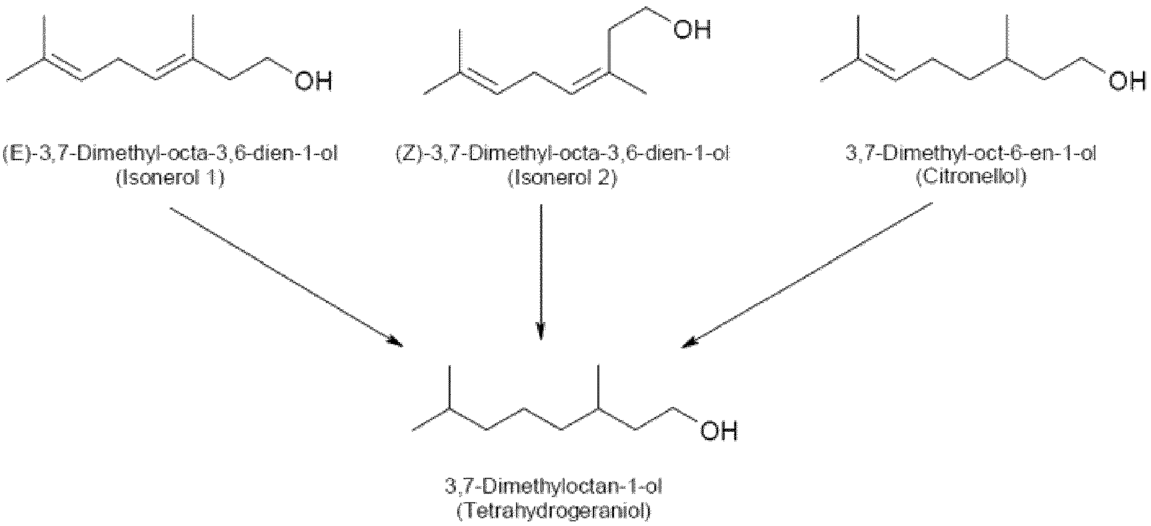

(E)-3,7-Dimethyl-octa-3,6-dien-1-ol (Isonerol 1)

(Z)-3,7-Dimethyl-octa-3,6-dien-1-ol (Isonerol 2)

3,7-Dimethyl-oct-6-en-1-ol (Citronellol)

3,7-Dimethyloctan-1-ol (Tetrahydrogeraniol)

The hydrogenation can be carried out on nickel-based catalysts, Raney-nickel and supported palladium catalysts. Suitable hydrogenation processes are described in DE 10160142 and EP 1318131.

(12) Preparation of 3,7-Dimethyloctanenitril:

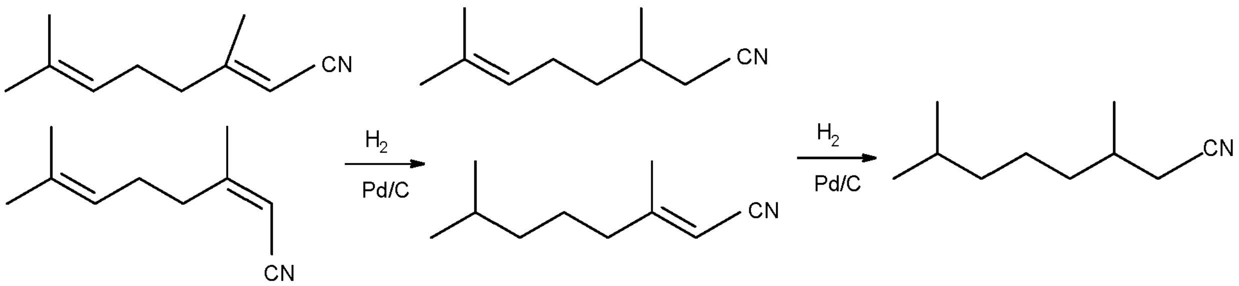


The hydrogenation can be carried out on a Pd/C catalyst in suspension at 20 to 100 °C and 10 to 30 bar, as for example described in WO 2006/106141.

(13) Hydrogenation of linalool to give tetrahydrolinalool:

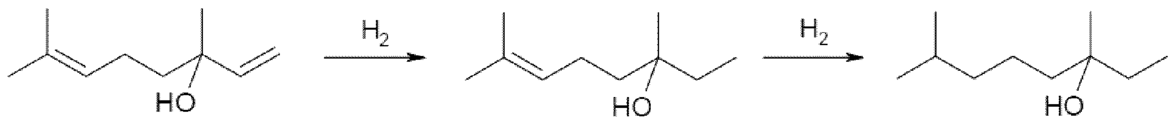


Linalool

Dihydrolinalool

Tetrahydrolinalool

The hydrogenation can be carried out on a Pd/C catalyst in suspension at 40 to 100 °C and 1 to 10 bar.

(14) Hydrogenation of pseudoionon to give tetrahydrogeranylacetone:

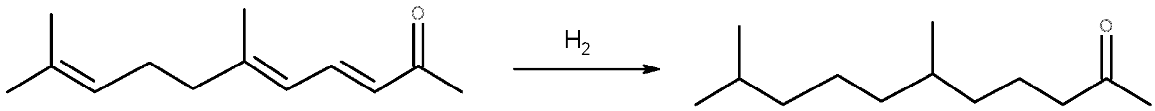


The hydrogenation can be carried out on a Pd/C catalyst in suspension at 60 to 125°C and for example 18 bar, as for example described in WO 2005/056508.

(15) Preparation of tetrahydronerolidol and isophythol (IP):

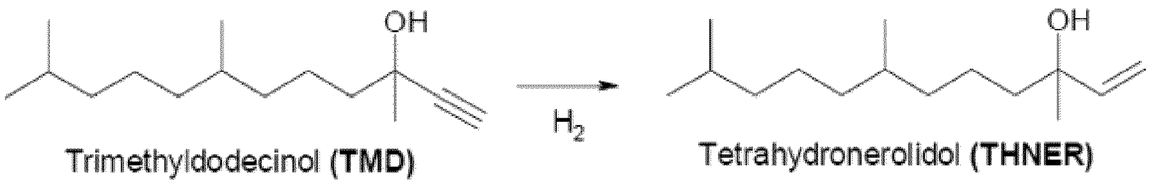


Trimethyldodecinol (**TMD**)

Tetrahydronerolidol (**THNER**)

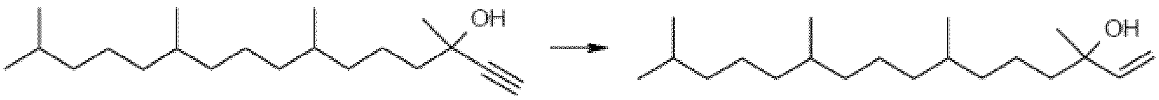

DIP

IP

The partial hydrogenation can be carried out on a palladium catalyst supported on a Kantal web. The hydrogenation is carried out continuously, CO can be added to control catalyst activity. A suitable catalyst and process is for example described in EP 1256560.

(16) Hydrogenation of tetrahydrofarnesylacetone (THFAC) to give hexahydrofarnesaylacetone (HEX):

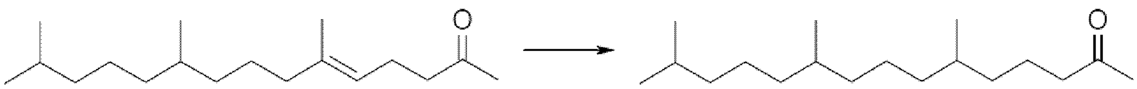

THFAC

HEX

The hydrogenation can be carried out as a fixed bed process on a Pd/$Al_2O_3$ catalyst at 80 to 150°C and 2 to 15 bar, as described in DE 19619013.

(17) Preparation of Lysmeral:

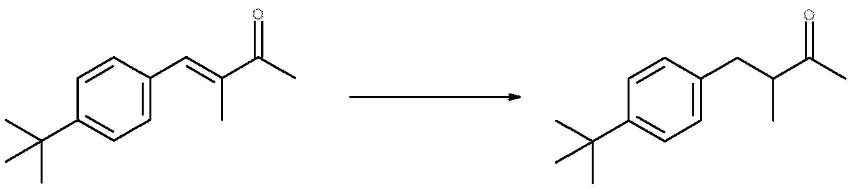

The hydrogenation can be carried out on a palladium catalyst supported on carbon or aluminum oxide at 10 to 150°C and 10 to 35 bar, as described in DE 102005049568.

(18) Preparation of rosenoxide from dehydrorosenoxide:

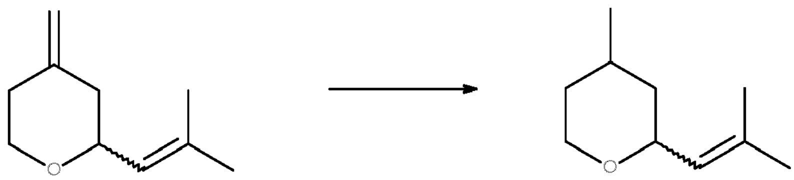

The selective partial hydrogenation can be carried out on ruthenium-based suspension catalysts, as for example described in US 2013/0109867.

(19) Preparation of dihydrorosane (DHR):

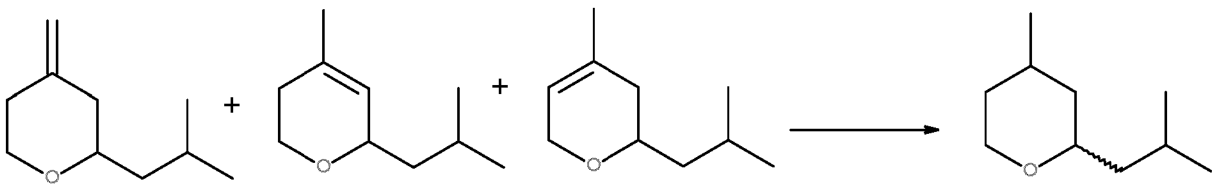

Hydrogenation of a mixture of unsaturated dihydropyranes (DHP 1,2,3) can be carried out on a Pd/C catalyst at 100 to 170 °C and 10 to 70 bar, as for example described in WO 2014/060345 and EP 2865676.

(20) Hydrogenation of cyclohexandimethanol divinylether to give cyclohexandimethanol diethylether ("Vertofruct"):

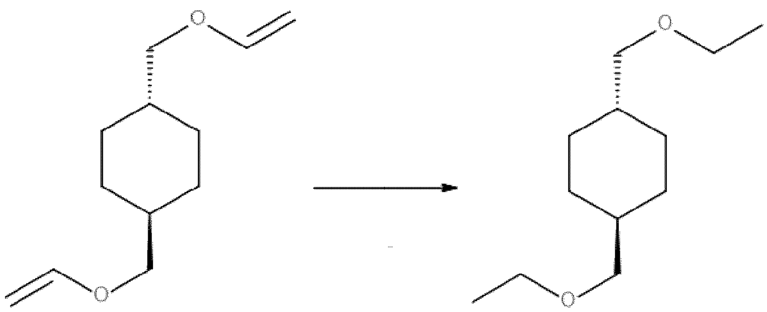

The hydrogenation can be carried out on a fixed bed $Pd/Al_2O_3$ catalyst at 30 to 150°C and 10 to 30 bar, as for example described in WO 2013/004579 and US 2014/0296579.

(21) Hydrogenation of unsaturated fatty acids to give saturated fatty acids or fatty alcohols:

Suitable processes are described in Ullmanns Encyclopedia of Industrial Chemistry, chapters "Margarines" and "Fats and Fatty Oils".

(22) Hydrogenation of cyclopentadiene (dimer) to give cyclopentene:

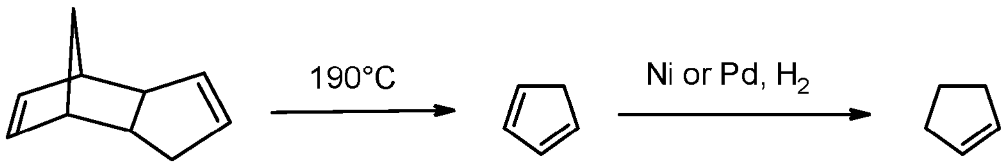


(23) Hydrogenation of alpha-ionon to alpha-dihydroionon

(24) Hydrogenation of beta-ionon to beta-dihydroionon

(25) Hydrogenation of pseudoionon to geranylacetate

(26) Hydrogenation of a compound of formula (1a) to a compound of formula (1b)

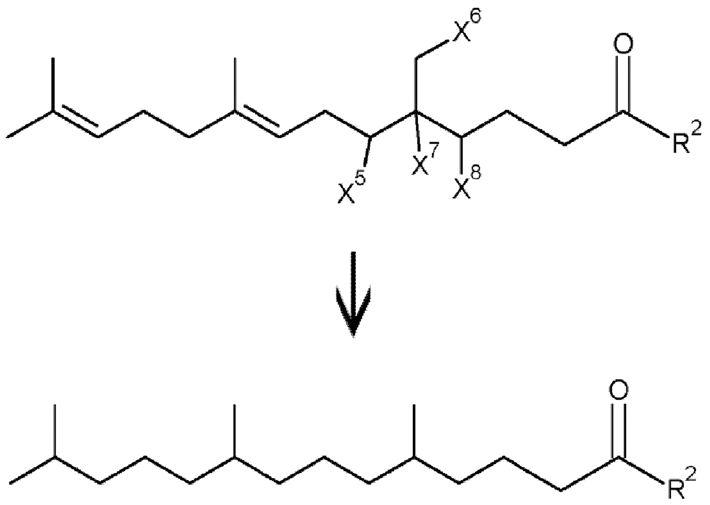

(1a)

(1b)

wherein

- $R^2$ is a $C_{1-4}$ alkyl radical, and preferably a methyl group, and
- either $R^5$ and $R^7$, $R^6$ and $R^7$, or $R^7$ and $R^8$ represent a double bond and the other two remaining radicals a hydrogen atom,

and any isomers or mixtures thereof.

Such a hydrogenation is preferably performed at around 50°C and around 1 MPa abs in the presence of a Pd/C suspension catalyst and further preferably in the presence of THF as solvent.

Thermal cleavage of the dimers with subsequent selective (partial) hydrogenation of the diene can be carried out according to the Zeon process or the Bayer process. The Zeon process is described in JP 2004175685 and JP 2001264592, the Bayer process is described in DE 2025411 and US 4048242.

The inventive processes of this application and the thus synthesized chemical compounds can be used in the synthesis of surfactants: Ethylenically unsaturated compounds can be reduced using the inventive hydrogen to yield the corresponding ethylenically saturated or partly saturated compounds. The reduction can for example be carried out by the methods described in WO2004035204, WO2011133037, WO2006121327 and the references cited therein. For example, triglicerides, fatty acids, fatty acid salts or fatty acid esters can be hydrogenated to their partly or fully saturated forms, using for example nickel-, platinum-, or palladium-catalysts in combination with the inventive hydrogen.

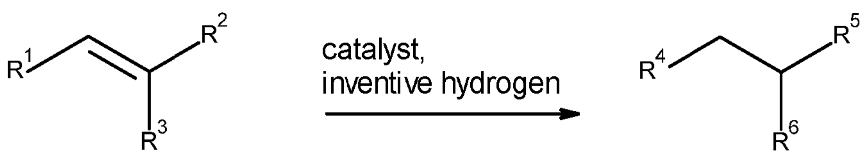

$R^1$ and $R^4$ are organic moieties, $R^2$, $R^3$, $R^5$ and $R^6$ can be organic moieties or hydrogen. If $R^1$ to $R^3$ contain ethylenically unsaturated moieties, these may or may not be reduced during the reaction step using the inventive hydrogen, depending on the reaction conditions chosen.

Examples for the reactions described above are shown in the table below.

| Reactant | Product | Conditions |
|---|---|---|
| Tallow fatty acid | Saturated tallow fatty acid | Ni catalyst, 200 bar hydrogen, 200 °C |
| Tallow fatty acid | Saturated tallow fatty acid | Ni/Cu catalyst, 100 bar hydrogen, 200 °C |
| Soy bean oil | Saturated soy bean oil | Ni catalyst, 100 bar hydrogen, 200 °C |

The ethylenically saturated or partly saturated compounds can be surface active compounds by themselves or can be used to produce surface active compounds, for example surfactants, for example by transferring them to the corresponding alcohols or amines followed b alkoxylation or by forming amides or esters with hydrophilic compounds.

The inventive saturated or ethylenically unsaturated aliphatic or cycloaliphatic compounds are characterized by the molar share of deuterium in the hydrogen bound in the compounds as a result of step (b) of the process according to the invention of ≤ 100 ppm, preferably in the range of from 10 to 95 ppm, more preferably in the range of from 15 to 90 ppm, most preferably in the range of from 20 to 80 ppm, especially in the range of from 30 to 75 ppm, based on the total content of said hydrogen.

Said low deuterium molar share is introduced by step (a) of the process according to the present invention. With the present invention environmentally friendly saturated or ethylenically unsaturated aliphatic or cycloaliphatic compounds and an environmentally friendly process for making the same, wherein said process uses as little fossil energy as possible, are provided.

As mentioned above, it is important that the origin of the hydrogen and down-stream compounds obtained by clean energy can be tracked in a reliable way.

Today, the majority of hydrogen is produced from fossil fuels by steam reforming of natural gas and other light hydrocarbons, partial oxidation of heavier hydrocarbons, and coal gasification. According to the invention, hydrogen obtained by electrolysis is obtained by using non-fossil energy sources. It is expected that the electrification (power generation) of fossil sources will be fully replaced by the generation of power by non-fossil resources in the near future. The downstream products based on hydrogen obtained by electrolysis can be distinguished by their deuterium molar share from saturated or ethylenically unsaturated aliphatic or cycloaliphatic compounds based on hydrogen prepared by petrochemical processes.

The present invention therefore relates to the use of the molar share of deuterium in downstream compounds based on hydrogen for tracing the origin, especially the energetic origin, of the hydrogen bound in the downstream compounds based on hydrogen, wherein the downstream compounds are saturated or ethylenically unsaturated aliphatic or cycloaliphatic compounds.

The present invention further relates to a process for tracing the origin, especially the energetic origin, of hydrogen bound in downstream compounds based on hydrogen by determining the molar share of deuterium in said downstream compounds based on hydrogen, wherein the downstream compounds are saturated or ethylenically unsaturated aliphatic or cycloaliphatic compounds.

Tracing is in the meaning of the present invention synonymous with tracking.

The origin is in the meaning of the present invention the preparation method of the hydrogen employed, especially electrolysis and/or the energetic origin, i.e. non-fossil energy sources. As mentioned above, it is expected that the electrification (power generation) of fossil sources will be fully replaced by the generation of power by non-fossil resources in the near future. Hydrogen made by electrolysis is in this case hydrogen of non-fossil origin. Examples for non-fossil power sources are mentioned above.

The inventive process for tracing the origin, especially the energetic origin, of hydrogen and downstream compounds mentioned above may be employed as a single tracing (tracking) method or in combination with further tracing (tracking) methods.

Suitable deuterium molar shares of the saturated or ethylenically unsaturated aliphatic or cycloaliphatic compounds are mentioned in the present application.

Furthermore, the products according to the process described herein can be converted to a product as described on page 18, line 29 to page 26, line 29 of European patent application No. EP24164893.0.

## Examples

### I Hydrogen Production

Experimental Setup and Method: Electrolysis cell design and Hydrogen production conditions

1) Polymer Electrolyte Membrane / Proton Exchange Membrane Electrolysis (PEM)

Electrolysis Cell: Water electrolysis was conducted with a circular commercial PEM electrolysis cell (model ZE 200, Sylatech Analysetechnik GmbH, 0.007 $m^2$ active area). The cell stack was sealed with O-rings and wrapped with heat insulation fabric for isothermal operating conditions. A Nation® 117 standard membrane (supplier DuPont, dry thickness 180 microns) was assembled by HIAT GmbH with catalytic active coating materials iridium (19 g/$m^2$) and platinum (8 g/$m^2$). Water distribution at the anode half-cell was realized with a titanium mesh. Before the polymer electrolyte membrane, a porous transport layer with sintered titanium fiber material is used for controlled flow while a porous graphite plate was situated on the cathode-side.

Experimental Conditions: Water with controlled temperature was supplied at constant flow of 9.5 g/h to the anode compartment. The cell pressure on cathode and anode side was controlled with PC valves. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic cell-water was re-cycled, whereas on the cathode the separated water was drained. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

2) Alkaline Electrolysis Cell (AEC)

Electrolysis Cell: Alkaline water electrolysis was conducted with a circular AEC electrolysis cell (model Electro MP Cell, supplier ElectroCell Europe A/S, 0.01 $m^2$ electrode area). As electrodes Nickel 2.4068 material was used and separated by a commercial standard Zirfon Perl UTP 500 membrane (open mesh polyphenylene sulfide fabric symmetrically coated with a mixture polymer / zirconium oxide; thickness 500 microns; 0.023 $m^2$ active area; supplier Agfa-Gevaert N.V.) in zero-gap cell configuration.

Experimental Conditions: Alkaline water (32 wt% potassium hydroxide, technical standard grade) with controlled temperature was supplied at constant flow of 27.8 kg/h to the anode and cathode compartment. The cell pressure on cathode and anode side was equalized via PC valve control. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic and cathodic cell-water was re-cycled. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

3) Anion Exchange Membrane / Alkaline Electrolyte Membrane Electrolysis (AEM)

Electrolysis Cell: The AEM experiments were executed in a commercial, fully automated 2.4 kW EL 4.0 cell supplied by Enapter GmbH, 10117 Berlin and a 1 wt% potassium hydroxide (standard grade) electrolyte solution.

Test station: As recommended by the supplier the EL 4.0 electrolyzer is run with a 1 wt% potassium hydroxide (technical standard grade) solution, hydrogen production at operating conditions (experimental conditions such as temperature and pressure settings see table) was 480 l/h at approx. 400 ml water consumption. The evolved hydrogen gas was treated with desiccant dryers, ensuring complete separation of moisture in the electrolysis gas flow to yield < 0,03 wt% water in the hydrogen gas stream. Small gas samples of the dehydrated cathodic hydrogen gas were taken from the continuous flow with an auto-sampler at regular intervals to conduct hydrogen gas analytics described below.

4) Solid Oxide Electrolysis Cell (SOE)

Solid Oxide cell stacks from Elcogen- Elco Stack (Elcogen OY, Vantaa 01510 Finland), were used and a E3000 unit was operated in reverse mode at 700°C and 35A electrolyzing current; Anode functional composition due to the supplier is NiO/YSZ. Cathode is of LSC type [La(Sr)CoO3]: The principle is also described in Novel high-performance solid oxide fuel cells with bulk ionic conductance dominated thin-film electrolytes - ScienceDirect (www.sciencedirect.com/science/article/pii/S037877531201097X); D. Stover et al, Journal of Power Sources; Volume 218, 15 November 2012, Pages 157-162.

The hydrogen stream was used with no further purification/dryer. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to

conduct hydrogen gas analytics described below.

The results of the hydrogen production are shown in Table 1.

Table 1

| Electrolyser type | Example I) PEM-electrolyser | Example II) PEM electrolyser | Example III) Alkaline electrolyser | Example IV) Alkaline electrolyser | Example V) AEM | Example VI) AEM | Example VII) Solid oxide |
|---|---|---|---|---|---|---|---|
| Water-type/deuterium content | VSMOW/155,76 ppm D | Selected* surface water/139 ppm D | VSMOV/155,76 ppm D | Selected* surface water/139ppm D | VSMOW/155,76 ppm D | Selected* surface water/139 ppm D | Selected* surface water/139 ppm D |
| Current density [A/cm2] | 1.35 | 1.35 | 0.8 | 0.8 | 1.6 | 1.6 | 0.78 |
| Applied voltage [V] | 1.9 | 1.9 | 2.15 | 2.15 | 1.9 | 1.9 | 1.35 |
| Operating temperature [°C] | 68 | 68 | 81 | 81 | 45 | 45 | 710 |
| Cell pressure [bar] | 23 | 23 | 25 | 25 | 35 | 35 | 1.3 |
| Voltage efficiency [%] | 57 | 57 | 53 | 53 | 66 | 66 | 81 |
| Electrical efficiency [kWh/kg H2] | 52 | 52 | 48.5 | 48.6 | 69 | 69 | 47 |
| Deuterium content in hydrogen [ppm] | 86 | 77 | 95 | 87 | 74 | 66 | 38 |

* Isar river surface water (Munich) collected in winter season January 2021.

Experimental Setup and Method: "D content (Deuterium content) in samples"

The following method descriptions apply for determination of the molar share of deuterium based on the total hydrogen content (Deuterium content) of gas and liquid samples. The isotopic H/D-share analysis is based on mass spectroscopy. Two different methods are used: method A for gas samples and method B for liquid samples.

For determination of the "D content in gas and liquid samples" it is of crucial importance not to contaminate the samples e.g. with ambient humidity or other ambient components containing hydrogen or deuterium. Therefore gas-tight materials and sealings must be used with clean sample containers to avoid any cross-contamination. Therefore, before filling and sealing a sample container it must be flushed at least 20 times the sample container volume with the gas or liquid stream to be analyzed. The same is valid for the experimental setup of the gas sampler and mass spectrometer. Utmost care must be taken to avoid cross-contamination e.g. via condensation of humidity. The analytical setup from sampling to mass spectrometry is validated with known reference samples.

Method A) Gas samples

Total Deuterium from HD and $D_2$ in hydrogen gas samples was determined via ultra-high resolution quadrupole mass spectrometry using a Hiden DLS-20 (Hiden Analytical Ltd., Warrington, Cheshire, UK) analyzer setup. The general method setup is described in C.C. Klepper, T.M. Biewer, U. Kruezi, S. Vartanian, D. Douai, D.L. Hillis, C. Marcus, Extending helium partial pressure measurement technology to JET DTE2 and ITER; Rev. Sci. Instrum., 87 (11) (2016); doi: 10.1063/1.4963713. For the hydrogen gas samples the threshold ionization mass spectrometry mode (TIMS) was used as described in S. Davies, J.A. Rees, D.L. Seymour; Threshold ionization mass spectrometry (TIMS); A complementary quantitative technique to conventional mass resolved mass spectrometry; Vacuum, 101 (2014), pp. 416-422; doi: 10.1016/j.vacuum.2013.06.004. Sensitivity is +/-1 ppm.

Method B) Liquid samples

Analysis of liquid samples (Saturated or ethylenically unsaturated aliphatic or cycloaliphatic compounds) was executed via isotope ratio monitoring gas chromatography/mass spectrometry (IRMS). Therefore a DELTA V PLUS CF-IRMS mass spectrometer was used. This mass spectrometer with magnetic sector with continuous flux DELTA V PLUS CF-IRMS is used to measure the isotopic ratio of D/H.

Measurement of D/H in a continuous He-flow mode needs the complete removal of low energy 4 He+ ions from the HD+ ion beam at m/z 3). The method is described in RAPID COMMUNICATIONS IN MASS SPECTROMETRY Rapid Commun. Mass Spectrom. 13, 1226-1230 (1999), W.A. Brandt et al. Sensitivity is within +/-3 ppm.

Method C) Analysis of isotope ratios in starting materials and products

Analysis of isotope ratios in starting materials and products was furthermore executed via SNIF-NMR related methods ("site-specific natural isotope fractionation studied by nuclear magnetic resonance") using high resolution $^2$H-NMR (Bruker Avance NEO 600 MHz NMR Spectrometer and Bruker Avance III HD 700 MHz NMR Spectrometer both equipped with TCI probes).

Principles of this technology are described in Gérard J. Martin, Serge Akoka, and Maryvonne L. Martin; SNIF-NMR Part 1: Principles; in Graham A. Webb (ed.), Modern Magnetic Resonance, Springer (2008) pp 1651-1658 as well as Maryvonne Martin, Benli Zhang, and Gérard J. Martin; SNIF-NMR Part 2: Isotope Ratios as Tracers of Chemical and Biochemical Mechanistic Pathways; in Graham A. Webb (ed.), Modern Magnetic Resonance, Springer (2008) pp 1659-1667 and Gérard J. Martin, Maryvonne L. Martin and Gérald Remaud; SNIF-NMR Part 3: From Mechanistic Affiliation to Origin Inference; in Graham A. Webb (ed.), Modern Magnetic Resonance, Springer (2008) pp 1669-1680.

II Production of saturated or ethylenically unsaturated aliphatic or cycloaliphatic compounds

Saturated or ethylenically unsaturated aliphatic or cycloaliphatic compounds are prepared in industrial scale synthesis processes as described above.

III Further examples

Compound B from compound A and compound D from compound C

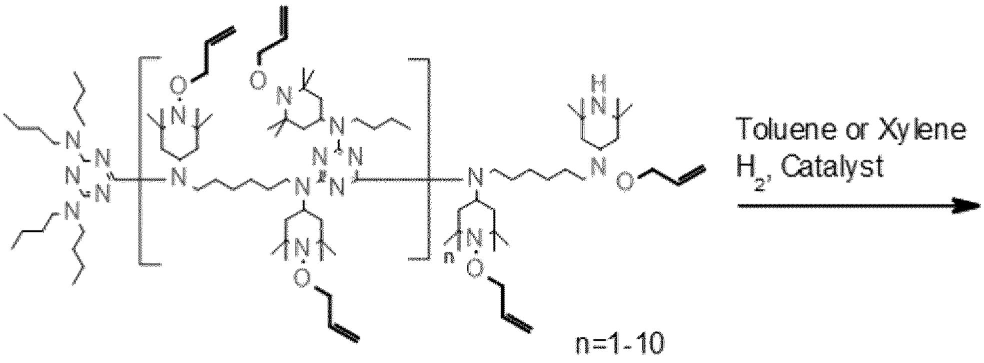


A

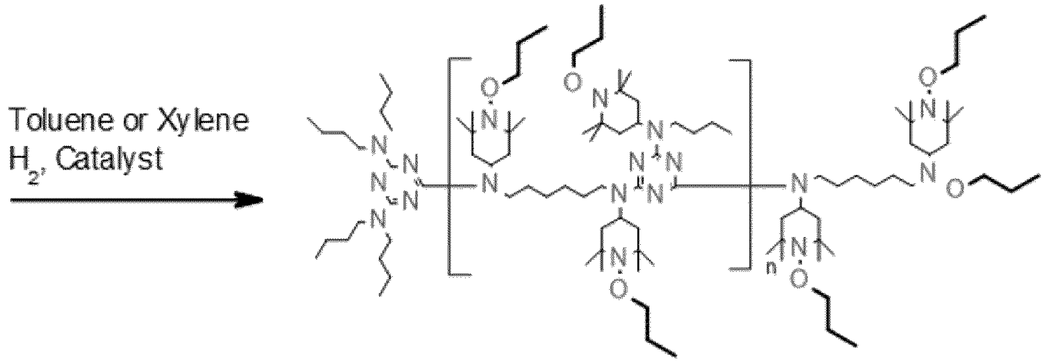


B

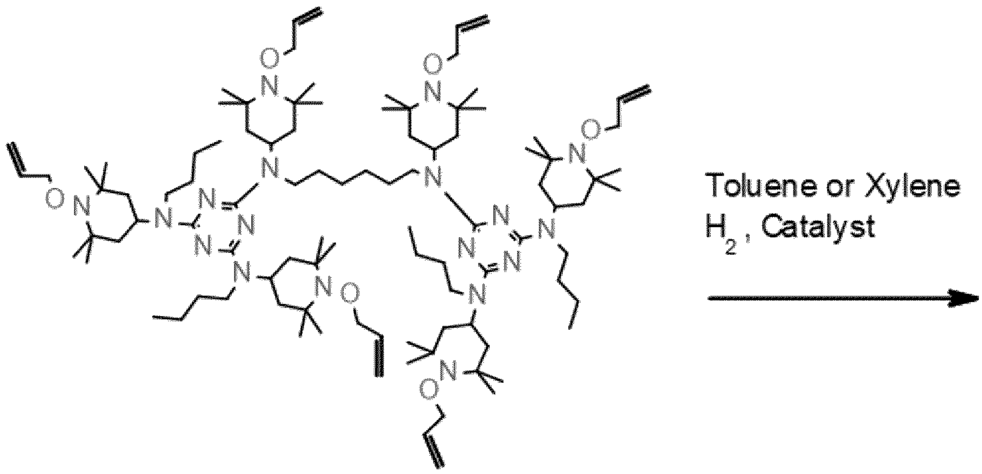


C

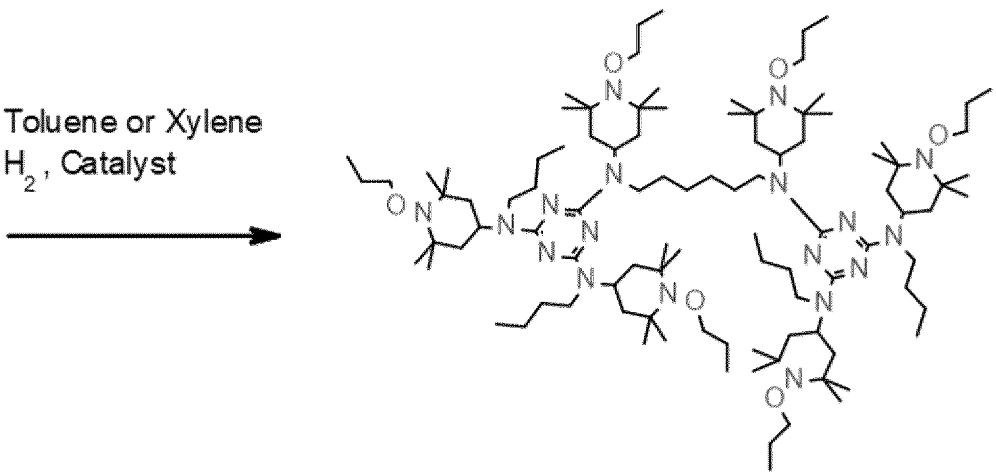


D

From the solution of the Compounds A or C prepared accordingly to previous steps, the solvent (xylene or toluene) was removed to get a final concentration between 45 and 60%. The mixture was hydrogenated in the presence of Pt or Pd catalyst supported on charcoal at 50 - 120°C, under pressure from 5 to 50 bar. After cooling the product was filtered from the catalyst to obtain the Compounds B or D.

## Claims

1. A process for the preparation of a saturated or ethylenically unsaturated aliphatic or cycloaliphatic compound comprising the following steps:

   (a) providing hydrogen with a molar share of deuterium ≤ 100 ppm, based on the total hydrogen content, by electrolysis of water using electrical power generated at least in part from non-fossil energy,
   (b) at least partially hydrogenating an ethylenically unsaturated compound to form the corresponding saturated compound, or at least partially hydrogenating an acetylenically unsaturated compound to form the corresponding saturated or ethylenically unsaturated compound.

2. The process of claim 1, wherein the electrical power is generated from wind power, solar energy, biomass, hydropower and geothermal energy.

3. The process according to claim 1 or 2, wherein hydrogen is provided by polymer electrolyte membrane water electrolysis.

4. The process according to claim 3, wherein hydrogen is provided by proton exchange membrane water electrolysis (PEMWE) or anion exchange membrane water electrolysis (AEMWE).

5. The process according to any one of claims 1 to 4, wherein the molar share of deuterium in the hydrogen provided in step (a) is in the range of from 10 to 95 ppm, preferably in the range of from 10 to 90 ppm, more preferably in the range of from 20 to 80 ppm, and most preferably in the range of from 30 to 75 ppm, based on the total hydrogen content.

6. The process according to any one of claims 1 to 5, wherein step (c) is carried out as heterogeneously or homogeneously catalyzed reaction.

7. The process according to any one of claims 1 to 6, wherein the ethylenically or acetylenically unsaturated aliphatic or cycloaliphatic compound to be hydrogenated in step (b) contains a total of from 2 to 20 carbon atoms and from 1 to 5, preferably from 1 to 3 carbon-carbon double and/or carbon-carbon triple bonds.

8. The process according to claim 7, wherein the ethylenically or acetylenically unsaturated aliphatic or cycloaliphatic compound to be hydrogenated in step (b) contains 1 to 5, preferably 1 to 3 functional groups selected from hydroxyl groups, carbonyl groups, nitrile groups, ether groups and amino groups.

9. The process according to claim 7 or 8, wherein the compound to be hydrogenated is an ethylenically unsaturated cycloaliphatic compound that can optionally contain ring heteroatoms selected from nitrogen, oxygen and sulfur atoms, preferably selected from nitrogen and oxygen atoms, and that contains 5, 6, 7, 8 or 12 ring carbon atoms, which can be optionally replaced by 1 to 3 ring heteroatoms.

10. The process according to any one of claims 7 to 9, wherein an ethylenically unsaturated aliphatic or cycloaliphatic compound containing two or more carbon-carbon double bonds is partially hydrogenated to an ethylenically unsaturated aliphatic or cycloaliphatic compound containing one carbon-carbon double bond.

11. The process according to any one of claims 7 to 9, wherein an acetylenically unsaturated aliphatic or cycloaliphatic compound is partially hydrogenated to the corresponding ethylenically unsaturated aliphatic or cycloaliphatic compound.

12. The process according to any one of claims 1 to 11, wherein the hydrogenation of the ethylenically unsaturated compound or acetylenically unsaturated compound is selected from

    (1) hydrogenation of butynediol to give butanediol;
    (2) hydrogenation of dimethylhexynediol to give dimethylhexanediol;
    (3) hydrogenation of maleic acid derivatives to give succinic acid derivatives;
    (4) hydrogenation of cyclododecadienone to give cyclododecanone;
    (5) hydrogenation of ethylhexenal to give ethylhexanal;
    (6) hydrogenation of 2-ethylhexenal to give 2-ethylhexanol;
    (7) hydrogenation of 2-propylheptenal to give 2-propylheptanol;

(8) partial and selective hydrogenations of cracker fractions;
(9) hydrogenation of citral to give citronellal;
(10) hydrogenation of isopulegol to give menthol;
(11) preparation of 3,7-dimethyloctan-1-ol by hydrogenation of (E)-3,7-dimethylocta-3,6-dien-1-ol (isonerol 1), (Z)-3,7-dimethylocta-3,6-dien-1-ol (isonnerol 2) and 3,7-dimethyloct-6-en-1-ol (citronellol);
(12) preparation of 3,7-dimethyloctanenitrile;
(13) hydrogenation of linalool to give tetrahydrolinalool;
(14) hydrogenation of pseudoionon to give tetrahydrogeranylacetone;
(15) preparation of tetrahydroneroliol and isophythol;
(16) hydrogenation of tetrahydrofarnesylacetone to give hexahydrofarnesaylacetone;
(17) preparation of lysmeral;
(18) preparation of rosenoxide;
(19) preparation of dihydrorosane;
(20) hydrogenation of cyclohexandimethanoldivinylether to cyclohexandimethanol diethylether;
(21) hydrogenation of unsaturated fatty acids to give saturated fatty acids or fatty alcohols;
(22) hydrogenation of cyclopentadiene to give cyclopentene.

**13.** Saturated or ethylenically unsaturated aliphatic or cycloaliphatic compound, obtainable by the process according to any one of claims 1 to 12.

**14.** Saturated or ethylenically unsaturated aliphatic or cycloaliphatic compound according to claim 13, wherein the molar share of deuterium in the hydrogen bound in the compound as a result of step (b) of the process according to any one of claims 1 to 12 is $\leq$ 100 ppm, preferably in the range of from 10 to 95 ppm, more preferably in the range of from 15 to 90 ppm, most preferably in the range of from 20 to 80 ppm, especially in the range of from 30 to 75 ppm, based on the total content of said hydrogen.

**15.** The use of the molar share of deuterium in downstream compounds based on hydrogen for tracing the origin, especially the energetic origin, of hydrogen bound in the downstream compounds based on hydrogen, wherein the downstream compounds are saturated or ethylenically unsaturated aliphatic or cycloaliphatic compounds.

**16.** A process for tracing the origin, especially the energetic origin, of hydrogen bound in downstream compounds based on hydrogen by determining the molar share of deuterium in said downstream compounds based on hydrogen, wherein the downstream compounds are saturated or ethylenically unsaturated aliphatic or cycloaliphatic compounds.

Europäisches Patentamt
European Patent Office
Office européen des brevets

# EUROPEAN SEARCH REPORT

Application Number
EP 24 20 7977

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/136097 A1 (SMAJLOVIC IVAN [RS]) 9 June 2011 (2011-06-09)<br>* the whole document *<br>----- | 15,16 | INV.<br>C25B1/04<br>C25B15/08<br>C01B4/00<br>C07B59/00<br>C07C4/22<br>C07C5/05<br>C07C7/163<br>C07C7/167<br>C07C29/17<br>C07C41/20<br>C07C45/62<br>C07C51/36<br>C07C211/00<br>C07D309/04<br>C10G45/00 |
| A | FELLOWS S K ET AL: "Availability of large quantities of low-deuterium hydrogen, and possible uses",<br>INTERNATIONAL JOURNAL OF HYDROGEN ENERGY, ELSEVIER, AMSTERDAM, NL,<br>vol. 6, no. 1, 1 January 1981 (1981-01-01), pages 67-71, XP025591252,<br>ISSN: 0360-3199, DOI: 10.1016/0360-3199(81)90098-7<br>[retrieved on 1981-01-01]<br>* the whole document *<br>----- | 15,16 | |
| X | US 2013/274511 A1 (FOLEY PATRICK [US] ET AL) 17 October 2013 (2013-10-17)<br>* the whole document *<br>----- | 1-16 | |
| X | WO 2021/212236 A1 (UNIV BRITISH COLUMBIA [CA]) 28 October 2021 (2021-10-28)<br>* the whole document *<br>----- | 1-16 | **TECHNICAL FIELDS SEARCHED (IPC)**<br>C25B<br>C01C<br>C01B<br>C07B<br>C07C |
| A,P | WO 2023/213713 A1 (BASF SE [DE]) 9 November 2023 (2023-11-09)<br>* claims 13, 14 *<br>----- | 15,16 | |

1

The present search report has been drawn up for all claims

| Place of search | Date of completion of the search | Examiner |
|---|---|---|
| Munich | 5 March 2025 | Ritter, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 20 7977

This annex lists the patent family members relating to the patent documents cited in the above-mentioned European search report. The members are as contained in the European Patent Office EDP file on
The European Patent Office is in no way liable for these particulars which are merely given for the purpose of information.

05-03-2025

| Patent document cited in search report | Publication date | Patent family member(s) | Publication date |
|---|---|---|---|
| US 2011136097 A1 | 09-06-2011 | NONE | |
| US 2013274511 A1 | 17-10-2013 | CA 2867698 A1 | 26-09-2013 |
| | | EP 2828231 A1 | 28-01-2015 |
| | | JP 6510400 B2 | 08-05-2019 |
| | | JP 6591518 B2 | 16-10-2019 |
| | | JP 2015510932 A | 13-04-2015 |
| | | JP 2018052975 A | 05-04-2018 |
| | | US 2013274511 A1 | 17-10-2013 |
| | | WO 2013142206 A1 | 26-09-2013 |
| WO 2021212236 A1 | 28-10-2021 | US 2023158459 A1 | 25-05-2023 |
| | | WO 2021212236 A1 | 28-10-2021 |
| WO 2023213713 A1 | 09-11-2023 | AU 2023266064 A1 | 14-11-2024 |
| | | CN 119156370 A | 17-12-2024 |
| | | EP 4519241 A1 | 12-03-2025 |
| | | KR 20250006960 A | 13-01-2025 |
| | | WO 2023213713 A1 | 09-11-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5068468 A
- EP 0177912 A
- WO 2012095777 A
- DE 10351396
- US 3371121 A
- EP 0333296 A
- DE 19500479
- WO 2021115813 A
- WO 9743242 A
- WO 9925678 A
- WO 2005030690 A
- WO 2008000757 A
- WO 2007006789 A
- DE 3530839
- US 20130237726 A
- CN 116041142
- EP 0011906 A
- EP 0885273 A
- WO 2019081628 A
- DE 10259858
- US 3459657 A
- DE 3709328
- DE 102007037785
- DE 2839474
- DE 19814879
- WO 2014167014 A
- US 20100249467 A
- WO 2009013192 A
- DE 10160142
- EP 1318131 A
- WO 2006106141 A
- WO 2005056508 A
- EP 1256560 A
- DE 19619013
- DE 102005049568
- US 20130109867 A
- WO 2014060345 A
- EP 2865676 A
- WO 2013004579 A
- US 20140296579 A
- JP 2004175685 B
- JP 2001264592 B
- DE 2025411
- US 4048242 A
- WO 2004035204 A
- WO 2011133037 A
- WO 2006121327 A
- EP 24164893


### Non-patent literature cited in the description


- **S. KUMAR** ; **V. HIMABINDU**. *Material Science for Energy Technologies*, 2019, vol. 2, 4442-4454
- **H. A. MILLER et al.** *Sustainable Energy Fuels*, 2020, vol. 4, 2114-2133
- **K. HARADA et al.** *International Journal of Hydrogen Energy*, 2020, vol. 45, 31389-31 395
- **H. SATO et al.** *International Journal of Hydrogen Energy*, 2021, vol. 46, 33 689-33 695
- Addition to Carbon-carbon Multiple bonds. March's Advanced Organic Chemistry: Reactions, Mechanisms and Structure. Wiley, February 2020
- Hydrogenation and Dehydrogenation. **C.STOCK**. Ullmann's Encyclopedia of Industrial Chemistry. 2023
- **RYLANDER**. Catalytic Hydrogenation in Organic Synthesis. Academic Press, 1979
- **RYLANDER**. Catalytic Hydrogenation over Platinum Metals. Academic Press, 1967, 59-120
- Hydrogenation and Dehydrogenation. **C.STOCK**. Catalysts'' in Ullmann's Encyclopedia of Industrial Chemistry. 2023
- **FAVRE-RÉGUILLION**. *Organic Process Research and Development*, 2016, vol. 20, 90-94
- **TANG et al.** *Applied Catalysis A, General*, 2004, vol. 273, 171-176
- **A. BORODZINSKI** ; **G.C., BOND**. *Catalysis Reviews*, 2006, vol. 48, 91-144
- Margarines. Ullmanns Encyclopedia of Industrial Chemistry
- Fats and Fatty Oils. Ullmanns Encyclopedia of Industrial Chemistry
- **D. STOVER et al.** *Journal of Power Sources*, 15 November 2012, vol. 218, 157-162
- **C.C. KLEPPER** ; **T.M. BIEWER** ; **U. KRUEZI** ; **S. VARTANIAN** ; **D. DOUAI** ; **D.L. HILLIS** ; **C. MARCUS**. Extending helium partial pressure measurement technology to JET DTE2 and ITER. *Rev. Sci. Instrum.*, 2016, vol. 87 (11)

- **S. DAVIES** ; **J.A. REES** ; **D.L. SEYMOUR**. Threshold ionization mass spectrometry (TIMS); A complementary quantitative technique to conventional mass resolved mass spectrometry. *Vacuum*, 2014, vol. 101, 416-422
- **W.A. BRANDT**. RAPID COMMUNICATIONS IN MASS SPECTROMETRY. *Rapid Commun. Mass Spectrom.*, 1999, vol. 13, 1226-1230
- SNIF-NMR Part 1: Principles. **GÉRARD J. MARTIN** ; **SERGE AKOKA** ; **MARYVONNE L. MARTIN**. Modern Magnetic Resonance. Springer, 2008, 1651-1658
- SNIF-NMR Part 2: Isotope Ratios as Tracers of Chemical and Biochemical Mechanistic Pathways. **MARYVONNE MARTIN** ; **BENLI ZHANG** ; **GÉRARD J. MARTIN**. Modern Magnetic Resonance. Springer, 2008, 1659-1667
- SNIF-NMR Part 3: From Mechanistic Affiliation to Origin Inference. **GÉRARD J. MARTIN** ; **MARYVONNE L. MARTIN** ; **GÉRALD REMAUD**. Modern Magnetic Resonance. Springer, 2008, 1669-1680